# EUROPEAN PATENT SPECIFICATION

(11) **EP 1 805 191 B1**
(45) Date of publication and mention of the grant of the patent: **14.12.2011**
(21) Application number: 04761492.0
(22) Date of filing: 14.09.2004
(51) Int. Cl.: C07F 9/12

(54) **Phosphorylated polyphenols as colour-stable agents**
Phosphorylierte Polyphenole als farbstabile Mittel
Polyphenols phosphorylés utilisés comme agents de couleur stable

(43) Date of publication of application: 11.07.2007
(73) Proprietor: Ajinomoto Omnichem S.A., B-1435 Mont-Saint-Guibert (BE)
(72) Inventor: VAN BRUSSEL, Willy, B-9000 GENT (BE); SCHELKENS, Geert, B-9230 WETTEREN (BE); RICQUIER, Patrick, B-8400 OOSTENDE (BE)
(74) Representative: pronovem
(86) International application number: PCT/BE2004/000131
(87) International publication number: WO 2006/029483

(56) References cited:
- WO-A-03/086414
- US-A- 3 433 805
- US-A- 4 255 336
- DATABASE WPI Section Ch, Week 199740 Derwent Publications Ltd., London, GB; Class B04, AN 1997-431464 XP002327640 -& JP 09 194493 A (AJINOMOTO TAKARA CORP) 29 July 1997 (1997-07-29)
- DATABASE WPI Section Ch, Week 198930 Derwent Publications Ltd., London, GB; Class D21, AN 1989-216489 XP002329391 -& JP 01 153695 A (SHOWA DENKO KK) 15 June 1989 (1989-06-15)
- DATABASE REGISTRY [Online] CHEMICAL ABSTRACTS SERVICE, COLUMBUS, OHIO, US; 3 January 2005 (2005-01-03), "1,3-Benzenediol, 5-[(1E)-2-[4-(phosphonooxy)phenyl]ethenyl] -bis(dihydrogen phosphate)" XP002329389 retrieved from STN Database accession no. RN: 807311-20-2
- DATABASE REGISTRY [Online] CHEMICAL ABSTRACTS SERVICE, COLUMBUS, OHIO, US; 18 November 2004 (2004-11-18), "1,3-Benzenediol, 5-[(1E)-2-[4-(phosphonooxy)phenyl]ethenyl] -bis(dihydrogen phosphate), trisodium salt" XP002329390 retrieved from STN Database accession no. RN: 783306-37-6

## Description

### Field of the invention

The present invention relates to a new class of polyphenols and their production. The products of the invention combine the excellent antioxidant properties of phenolic compounds with a very good colour-stability.

This new class of polyphenols is particularly suitable for use in applications where colour-stability is of great importance.

### Background of the invention

Polyphenols exhibit a lot of advantages over simple phenolic compounds. More complex polyphenols are for instance known to be much better complexing agents for metals and proteins than simple phenolic compounds. They are also more active as antibacterials, antiallergens, antioxidants and radical scavengers. Consequently, they are the compounds of choice for applications in these fields. There are, however, some drawbacks, mainly related to stability such as colour-stability.

Compounds containing aromatic hydroxyl functions such as polyphenols are often used as antioxidants, or as radical scavengers to protect products and materials against various types of deterioration. Textiles are often treated with phenolic compounds such as tannins in order to protect polymeric fibres against oxidation induced by light, air and/or metal ions (Litherhand and Young (1983), Journal of the Society of Dyers and Colourists 99:201-207).

Polyphenols may also be used to protect nylon carpets from staining (Inside textiles, vol.7, p.1 (1986)), or to treat textiles with tannins that may act as an antiallergenic or an antibacterial. Tannins are also used to treat leathers. They are used for instance in the manufacture of lightly coloured leather for high quality textiles or car interiors.

A major problem implied, with the use of compounds containing aromatic hydroxyl functions is the possibility of yellowing or discolouration of the treated materials. This represents a major drawback for the use of phenolic compounds. (Litherhand and Young (1983), Journal of the Society of Dyers and Colourists 99:201-207)

Polyphenols are susceptible to degradation under influence of heat or light or by reaction with reactive species such as NOₓ, leading to discolouration of the material containing the polyphenol. Degradation may also take place during storage of the product or material. These degradation processes affect both the quality of the products or materials and the activity of the polyphenolic compounds present in these products or materials.

Various methods for the phosphorylation of aromatic alcohols are reviewed in Houben-Weyl, Methoden der Organischen Chemie, Organische Phosphorverbindungen, Vierte Auflage, Teil II, Seite 143-586 (1964).

A number of possible reagents is described herein. Phosphate esters for instance can be prepared by reacting an alcohol with phosphoric acid or anhydrous forms of phosphoric acid such as metaphosphate, pyrophosphate, phosphorous pentoxide or polyphosphoric acid. These reagents are used to phosphorylate aliphatic alcohols. Aromatic alcohols are less nucleophile, and hardly react with these reagents. The reactions that are described here are often performed in strongly acidic medium incompatible with many other functionalities.

This review also describes the use of phosphorous oxychloride and the use of more complex phosphoryl halides The reactions are always carried out in solvents, and an organic base is used to catalyse the reaction and to neutralize the hydrochloric acid formed. The most general system is to use pyridine as the base. The use of solvents and organic bases such as pyridine or alkylamines is not preferred from an environmental point of view, and is far from ideal for products to be used in food and medical applications.

There is not much literature on the phosphorylation of phenolic compounds. For example, gallic acid 4-monophosphate is described in Isoyama et al. (1968, Nichidai Igaku Zasshi 27:270-272). Tocopherol (vitamin E) phosphate and analogues of tocopherol phosphate are claimed as intermediates in the synthesis of ascorbic acid-phosphate-tocopherol (European Patent Application EP-A-0306904). Phosphorylation of 1-(2,4,6-trihydroxyphenyl)-3-(4'-hydroxyphenyl)-2-propanone and a number of analogues is disclosed in published U.S. Patent Application 2003/0162753. All preparations involve the use of solvents and organic bases.

The addition of phosphoryl chloride (POCl₃) to a mixture of an alcohol and a sugar to obtain an alcohol-OP(O)O-sugar co-phosphorylation product, where the alcohol may be pyrogallol, hesperitine, anthrachinon, is disclosed in GB 773,495. This co-phosphorylation product is prepared by mixing the reagents and without control over the formation of the aromatic phosphate. There is no indication whatsoever of phosphorylated alcohols being present as intermediate product in this reaction process.

JP8027318 *(applicant Denki Kagaku Kogyo KK)* discloses flame-retardant compositions that contain among others boron-containing polyphenol polymers. The latter are prepared by reacting polyphenolic compounds with phosphoric acid, phosphoric ester or a phosphoryl chloride, boric acid, boron oxide, or boric ester. The polyphenolic compounds disclosed herein are synthetic polymers of phenol, especially phenol resins and phenolic ether compounds.

JP9194493 (*applicant Ajinomoto Takara Corp KK*) discloses tannin compositions that are prepared by blending for instance a tannin or polyphenol with a phosphoric acid or a phosphoric acid derivative and with a number of other compounds such as thickening agents and stabilisers. The composition has been developed for use in car leather treatment to avoid discolouration of the leather. However, it is not studied in this publication whether phosphorylated compounds have been formed nor what the final composition looks like.

The phosphorylation methods described in literature mostly make use of phosphorous oxychloride in combination with organic solvents and bases, mostly pyridine. The reaction conditions are most often severe.

### Aims of the invention

The present invention aims to avoid drawbacks of the state of the art and provides a new class of stable polyphenolic compounds with many different application possibilities. This new class of compounds should combine advantageous properties of polyphenols such as tannins, for instance their excellent antioxidant activity, with better stability, for instance a better colour-stability.

There is a need for active, stable i.e. long-lasting products that are particularly useful in for instance food, textile and medical applications.

Yet another aim of the invention is to provide a very simple, cheap and efficient production process that avoids the use of noxious solvents and reagents.

### Summary of the invention

A first aspect of the present invention concerns active and stable polyphenolic compounds that consist of phosphate esters of polyphenols as defined in the appended claims. The phosphorylation of these compounds, preferably via the production process described in the present invention, stabilises these compounds against deterioration while significant activity is retained. With a phosphate ester of a phenolic compound is meant in the present context an ROP(O) (OH)₂ product, an ROP(O) (OH)OP(O) (OH)₂, or an ROP(O) (OH)OP(O) (OH)OP(O) (OH)₂ product and/or a metal salt of one of these; with R representing an organic molecule derived from polyphenols which may contain extra phosphate ester groups.

In the polyphenolic compounds such as the tannin compounds of the invention, part or at least a part of the phenolic hydroxyl groups are phosphorylated, thus preventing rapid degradation of the phenolic antioxidant. The phosphorylated products of the invention will contain at least one phosphate group. For some applications, full phosphorylation of all hydroxyl groups or full phosphorylation of all aromatic hydroxyl groups may be of interest.
In case colour stability is the most important, a high degree of phosphorylation would be useful, in case the activity prevails, a lower degree of phosphorylation would be better. The degree of phosphorylation required to obtain satisfactory colour stability varies from one substrate to another, and from one application to another.

The phosphorylated polyphenolic compounds can be divided into condensed and hydrolysable tannins. Condensed tannins are polyphenols containing at least two aromatic rings, each substituted with at least one hydroxyl function, and with at least two of these aromatic rings attached to each other by one or more single or double carbon-carbon bonds and/or an ether linkage of the form (CH₂)ₐO(CH₂)_{b} with a and b independently from each other between 0 and 3. Typical product categories are flavonoids, stilbenes and phloroglucinols.

An example of a tannin of the invention is a phosphorylated hydrolysable tannin with at least one covalently bound phosphate group selected from the group consisting of OP(O) (OH)₂, OP(O) (OH)OP(O) (OH)_{2,} OP(O) (OH)OP(O) (OH)OP(O) (OH)₂ and a metal salt of such phosphate groups. An hydrolysable tannin is a polyphenol composed of a central nucleus such as glucose or quinic acid esterified with a number of molecules such as gallic, digallic, trigallic and ellagic acid or combinations thereof (see infra).

Advantageously, the products of the invention exhibit excellent colour stability and do not induce yellowing or discolouration of materials treated with these products.

The compound to be phosphorylated, i.e. the polyphenol or the tannin, may be isolated from natural sources.

Further, the present invention relates to a phosphorylated condensed tannin or a salt thereof) corresponding to the formula: 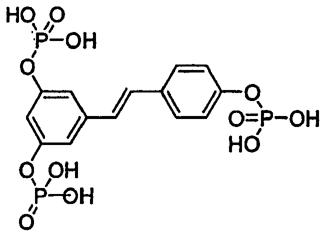 This compound is named resveratrol triphosphate. The salt may be any alkali metal salt in a ratio of 1-6 alkali metal atoms per molecule of resveratrol triphosphate. A preferred embodiment is the trisodium salt of resveratrol triphosphate:

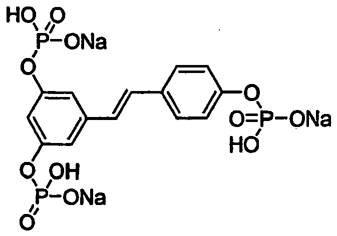

A suitable phosphorylation method comprises the steps of reacting a phosphoryl chloride with a polyphenol in aqueous medium using inorganic bases. In a preferred embodiment this aqueous medium is water. The inorganic base can be a metal hydroxide, a carbonate, a phosphate and/or ammonia. Most preferred is sodium hydroxide. The base can be added prior and/or during addition of the phosphorylating agent, i.e. the reagent.

The base is used to increase the pH of the reaction mixture to values between 5 and 13 depending on the acidity of the hydroxyl function. Preferably, the pH of the reaction mixture is kept between 7 and 12.

The phosphoryl chloride preferably is represented by the general formulas:

RₙPOX₃₋ₙ (IV) or RᵥPX_{w} (V)

Wherein n = 0, 1 or 2, v + w = 3 or 5, X is a chloride, bromide or iodide and R is an alkoxide with an alkyl chain of 1-8 carbon atoms, or O-Phenyl or O-benzyl. In an embodiment according to the invention, the phosphoryl chloride is phosphorous oxychloride (POCl₃).

The phosphoryl chloride that is used as phosphorylating agent preferably is applied in a ratio varying from 0.5 equivalent to over 25 equivalents per mole of phenolic hydroxyl compound, depending on the structure of the phenolic antioxidant, and more specifically on the amount of hydroxyl groups present in the molecule. The degree of phosphorylation can be controlled using high-pressure chromatography for instance.

After the phosphorylation step, the reaction mixture may be acidified. Preferably, a pH of 2 to 7 is obtained by the addition of mineral acids. The product obtained in aqueous solution can be used as such, can be concentrated by distillation or other means known to those skilled to the art and/or can be dried. The phosphorylated product according to the invention may be present as such or in a more or less purified form.

The compound to be phosphorylated may be a condensed or hydrolysable tannin.

The condensed tannin may be a condensed tannin represented by or corresponding to the general formula: 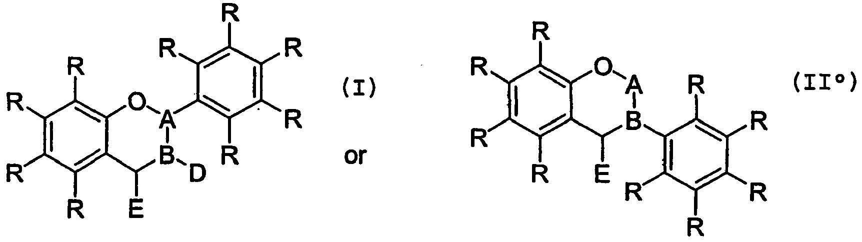 wherein:
- A and B are carbon atoms connected by a single or by a double bond
- D is hydrogen, hydroxide, or a hydroxide esterified with gallic acid or ellagic acid
- E is hydrogen, hydroxide, O-glucose or another condensed tannin corresponding to Formula (I) or (II)
- R is hydrogen, hydroxide, O-glucose, an O-alkyl group containing 1-3 carbon atoms or a phosphate group selected from the group consisting of OP(O) (OH)₂, OP(O) (OH)OP(O) (OH)₂, OP(O) (OH)OP(O) (OH)OP(O) (OH)₂ and a metal salt of such phosphate groups, with at least one of R being a phosphate group.

The compound to be phosphorylated may also be a hydrolysable tannin that is a polyphenol composed of a central nucleus such as glucose or quinic acid esterified with a number of molecules such as gallic, digallic, trigallic and ellagic acid or combinations thereof (see infra).

In an embodiment according to the invention the phosphorylated tannins described in the appended claims are used in a method to flock textiles to obtain a product with improved colour stability and attractive economical advantages

### Description of the figures

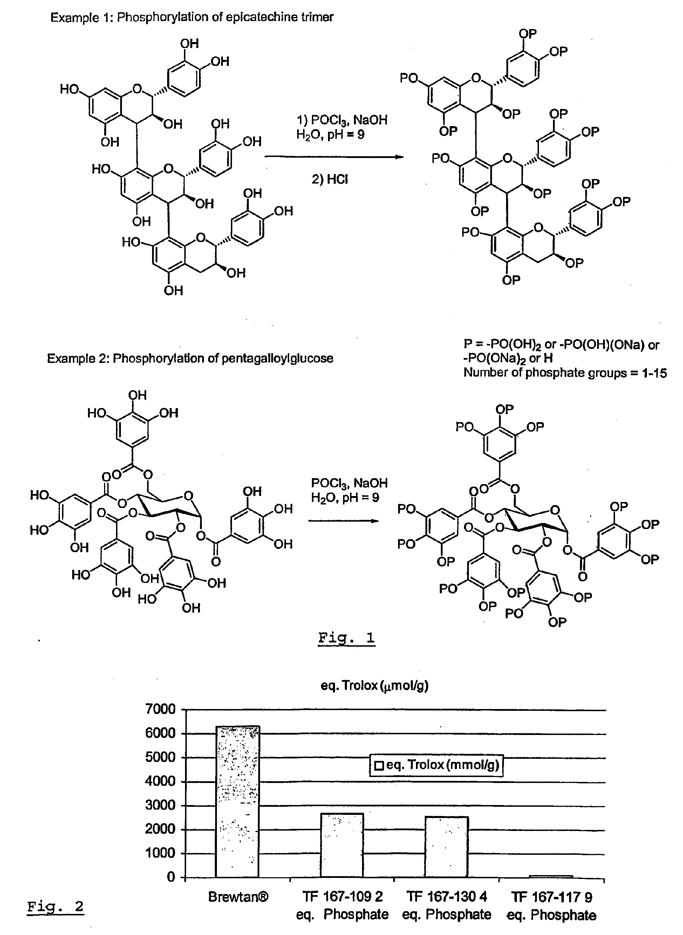

Figure 1 shows a general reaction scheme for both condensed and hydrolysable tannins.

Figure 2 shows the actual antioxidative capacities of the phosphorylated polyphenols as measured by the Trolox test. The acute antioxidative properties of Chinese gallnut tannin were studied using Brewtan(R), which was phosphorylated using 2, 4 and 9 equivalents of POCl₃. When 2 and 4 equivalents are used, part of the original acute activity remains. If 9 equivalents are used, which is a little more than 1 phosphate group per gallic acid group in the molecule, almost all acute activity is suppressed.

The invention will now be described in further detail in the following examples and embodiments by reference to the enclosed drawings, which are not in any way intended to limit the scope of the invention as claimed.

### Detailed description of the invention

The present invention provides a novel class of polyphenolic and/or phenolic compounds. The phosphate esters of the hydrolysable tannins, and condensed tannins combine the interesting properties of polyphenols in general with excellent colour stability and with improved resistance to wearing. The phosphorylated compounds of the invention are much less prone or much less susceptible to degradation or deterioration of the product under the influence of heat, light or reaction with gases such as NOₓ.

The degree of phosphorylation should be sufficient to obtain a satisfying product stability, and thus also depends on the requirements for the application.

The novel class of compounds, defined as phosphorylated polyphenols, consists of phosphorylated condensed tannins as defined in claims 1 and 2 and phosphorylated hydrolysable tannins and phosphorylated flavonoids as defined in claim 1.

### Phosphorylated condensed tannins

Condensed tannins are polyphenols containing at least two aromatic rings, each substituted with at least one hydroxyl function, and with at least two of these aromatic rings attached to each other by one or more single or double carbon-carbon bonds and/or an ether linkage of the form (CH₂)ₐO(CH₂)_{b} with a and b independently from each other between 0 and 3. Typical product categories are flavonoids, stilbenes and phloroglucinols.

Phosphorylated condensed tannins are condensed tannins where at least one of the hydroxyl functions of the molecule is phosphorylated.

It should be noted that the terms "hydrolysable and condensed tannins" refer to compounds as disclosed in the standard work " Chemistry of vegetable tannins" by E. Haslam, Ed. Academic Press, London, 1966.

An important category of phosphorylated polyphenols are the aromatic phosphate esters of condensed tannins. With an aromatic phosphate ester is meant in the present context either an AroP(O) (OH)₂ product, an ArOP(O) (OH)OP(O) (OH)₂, or an. ArOP(O) (OH)OP(O) (OH)OP(O) (OH)₂ product and/or a metal salt of one of these. With Ar is meant a condensed tannin in this particular case, more in particular a flavonoid as described below.

A flavonoid can be defined as a compound with a structure represented by or corresponding to the general formulas (I) or (II): 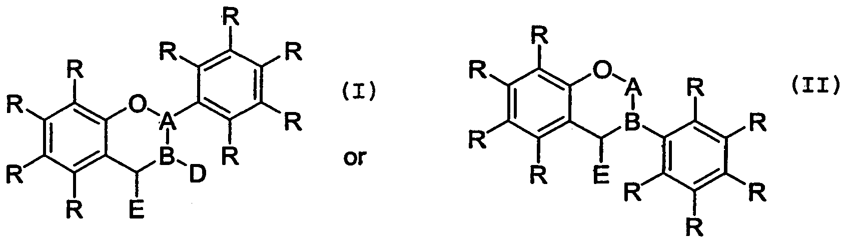 wherein:
- A and B are carbon atoms connected by a single or by a double bond
- D is hydrogen, hydroxide, or a hydroxide esterified with gallic acid or ellagic acid
- E is hydrogen, hydroxide, O-glucose or another phosphorylated flavonoid corresponding to Formula (I) or (II)
- R is hydrogen, hydroxide, O-glucose, an O-alkyl group containing 1-3 carbon atoms or a phosphate group selected from the group consisting of OP(O) (OH)₂, OP(O) (OH)OP(O) (OH)₂, OP(O) (OH)OP(O) (OH)OP(O) (OH)₂ and a metal salt of such phosphate groups, with at least one of R being a phosphate group.

In one embodiment the present invention relates to phosphorylated condensed tannins according to Formula (III) 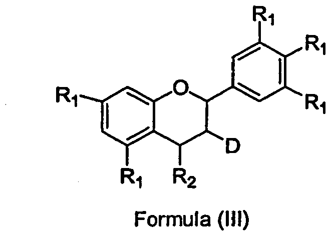 wherein
- R₁ is hydrogen, hydroxide, O-glucose, an O-alkyl group containing 1-3 carbon atoms or a phosphate group selected from the group consisting of OP(O) (OH)₂, OP(O) (OH)OP(O) (OH)₂, OP(O) (OH)OP(O) (OH)OP(O) (OH)₂ and a metal salt of such phosphate groups, with at least one of R₁ being a phosphate group.
- D is hydroxide, O-glucose or a galloyl residue with 0-3 phosphate groups as defined for R₁
- R₂ is a hydrogen or another component of Formula (III)

Typical product categories according to this formula are flavanoles and flavan-3,4-diols. Such flavonoids can occur in nature as much more complicated structures, for example as dimers or oligomers of the general structure defined above, or as even more complex derivatives thereof.

Typical examples of flavanols are catechine, epicatechine, gallocatechine, epigallocatechine, dimers of catechine or epicatechine, oligomers and polymers of catechine or epicatechine where the monomers are connected via a C-C bond. The oligomers are known as proanthocyanidines, the polymers as condensed tannins. Further groups of compounds belonging to this category are cyanidins, anthocyanidins and procyanidins.

Yet another group of condensed tannins are the phloroglucinols, consisting of pyrogallol dimers, oligomers and polymers, where the pyrogallol moieties are connected via ether bonds or via C-C bonds between two aromatic carbon atoms.

All these compounds defined as condensed tannins may be further esterified with acids such as ellagic acid or with gallic acid.

Ellagic acid itself can also be considered as a condensed tannin.

Mixtures of more than one of these compounds may also be used. Such a mixture may be obtained by phosporylation of a complex natural product extract.

Examples of natural extracts containing condensed tannins include tannin extracts from trees such as Eucalyptus, Acacia species or Quebracho, or from other plant sources, such as grape seed extracts, green tea extracts, black tea extracts, cocoa extracts, wine polyphenols, tannins from fruit or vegetables such as Persimmon or Kaki tannin, grapes, berries, citrus fruits or soy beans, or tannins from herbs and spices such as rosemary. Such natural product may be an extract from the wood and bark of Acacia sp., Schinopsis sp. (Quebracho tannin), Eucalyptus sp., Castanea sp., Quercus sp., Rhizophora sp., Picea sp., Pinus sp. or Larix sp.

Condensed tannins may also be synthetically prepared by coupling reactions between phenolic compounds. These compounds are known under the general name Syntans.

All these condensed tannins and flavonoids may be partially or fully phosphorylated to provide a novel class of phosphorylated condensed tannins.

### Phosphorylated hydrolysable tannins

A second category of phosphorylated polyphenols according to the invention are the aromatic phosphate esters of hydrolysable tannins. With an aromatic phosphate ester is meant in the present context either an ArOP(O) (OH)₂ product, an ArOP(O) (OH)OP(O) (OH)₂, or an ArOP(O) (OH)OP(O) (OH)OP(O) (OH)₂ product and/or a metal salt of one of these. With Ar is meant a hydrolysable tannin in this particular case.

Hydrolysable tannins are complex molecules occurring in natural products, composed of a central nucleus with hydroxybenzoic acids or hydroxycinnamates esterified onto the central nucleus or esterified onto an aromatic hydroxyl function of the molecule. This last type of bond is known as a depsidic bond. Examples of products containing one or two depsidic bonds are digallic acid and trigallic acid. Examples of these hydroxybenzoic acids are gallic acid and ellagic acid. Examples of hydroxycinnamic acids are caffeic acid, ferulic acid or synaptic acid.

Hydrolysable, naturally occurring tannins all contain such acids or a mixture of these acids esterified on glucose, on glycerol, on quinic acid, on shikimic acid, on a carbohydrate or a sugar in general.

The most abundant central nuclei are glucose such as in tannin from Chinese gallnuts and Aleppo nuts, and quinic acid such as in Tara tannin. The most abundant organic acids esterified onto these central nuclei are gallic acid and/or ellagic acid. Hydrolysable tannins composed with ellagic acid are known as ellagitannins.

Hydrolysable tannins based on glucose and gallic acid are typically composed of gallic acid and its oligomers, monogalloylglucose, di-, tri-, tetra-, penta-, hexa-, hepta-, octa-, nona-, deca-, undeca-, and dodecagalloylglucose, and smaller quantities of ellagitannins. Higher substitutions may also occur, but are rare. They are a mixture of the mentioned compounds in a ratio largely dependent on the plant origin.

A preferred class of phosphorylated compounds are the phosphate esters of 1,2,3,4,6-pentagalloylglucose, with 1-15 phosphate groups present on the molecule, depending on the application envisaged.

Preferably, the phosphorylated pentagalloylglucose contains between 2 and 10 phosphate groups.

Hydrolysable tannins are typically obtained by extraction of plant materials such as Chinese gall nuts, Bengal Kino, Aleppo nuts, Sumac tannin, Turkish tannin, Tara tannin, Acer tannin. Hydrolysable tannins are present in all plants, and may also be extracted from other plant sources.

The extracts known under the trade names Brewtan^{®}, TANAL, TANEX, FLOCTAN and TEXTAN are examples of preferred hydrolysable tannin extracts.

Alternatively, hydrolysable tannins may also be composed of dimers and oligomers of hydroxybenzoic acids such as for example the dimeric ester digallic acid, ellagic acid, trigallic acid and higher oligomers.

Hydrolysable tannins may also consist of condensed tannins (see supra) serving as the central nucleus on which gallic acid or ellagic acid is esterified.

Combinations of tannins with different properties may be used to improve the performance of the products.

Both hydrolysable and condensed tannins and examples thereof are well described in the standard work "Chemistry of vegetable tannins" by E. Haslam, Ed. Academic Press, London, 1966.

Other derivatives found in nature include esters and ethers of gallic acid and/or ellagic acid with simple alcohols such as methanol or ethanol. These may also combine to more complex structures, in the same way as described above.

### Vegetable sources of hydrolysable and condensed tannins

It will also be recognized that the useful compounds or groups of compounds can be used in substantially pure form, i.e., at a purity of 80% or greater, or they can be provided as a part of a plant extract. Virtually every plant contains some form of hydrolysable and condensed polyphenol, but there are certain plants or plant extracts that are recognized as being particularly rich sources of polyphenols. Examples of plants which may produce extracts useful in the compositions include plants of the genera: *Gingko, Lespedeza, Passiflora, Silybum, Citrus, Hamamelis, Thymus, Chamaemelum, Achillea, Equisetum, Sophora, Fagopyrum, Eucalyptus, Sambucus, Betula, Vitis, Pinus, Crataegus, Quercus, Ratanhia, Lythrum, Acacia, Cupressus, Vaccinium, Ribes, Centaurea, Rosa, Hibiscus, Camellia, Malva, Podophyllum, Schizandra, Gaiacum, Theobroma, Arctostaphylos, Glycine, Cynara, Rosmarinus, Orthosiphon, Solidago, Lithospermum, Curcuma, Aesculus, Melilotus, Ammi, Hieracium, Angelica,* and *Asperula.* In particular, it is well known that particularly rich sources of polyphenols include red wine, grape juice, grape skins, grape seeds, blueberries, persimmon, eucalyptus, cocoa, green tea, black tea, white tea, pomegranate, and Chinese gallnut. Thus, when referring to phosphorylated polyphenols in the present specification and claims, this phrase is intended to cover not only isolated compounds that have been phosphorylated, but also extracts of plant materials containing polyphenols, which extracts have also been subjected to the phosphorylation procedure, thereby phosphorylating the polyphenols contained therein.

### Phosphorylation: degree and preferred P-groups

The degree of phosphorylation in the condensed and hydrolysable tannins should be sufficient to obtain a satisfying product stability, and thus also depends on the requirements for the application.

The compounds of the present invention contain a number of phosphate groups going from one phosphate group to full phosphorylation of all aromatic hydroxyl functions present in the molecule. The ideal degree of phosphorylation depends on the stability required in the application. Some applications require full phosphorylation.

Typically, the ideal number of phosphate groups is one per aromatic ring containing two or more hydroxyl functions.

The phosphate group can be OP(O)(OH)₂, OP(O) (OH)OP(O) (OH)₂, OP(O) (OH)OP(O) (OH)OP(O) (OH)₂, and/or can be a metal salt of any one of these groups
Preferably, the phosphate ester is an ester such as OP(O) (OH)₂ or a metal salt of this phosphate ester, preferably a metal of Group Ia or Group IIa, such as lithium, sodium, potassium calcium or magnesium.

Most preferred is the phosphate ester OP(O) (OH)₂ or a mono- or disodium salt or a calcium salt of this particular ester.

The polyphenols suitable for the application are condensed or hydrolysable tannins, or compounds with a structure similar to these tannins. This class of compounds is known to exhibit excellent antioxidant activities and possesses better antioxidative properties than eg. gallic acid or alkylgallates due to their molecular weight and their complex structure.

### Dimeric or trimeric phosphates, di- or triphosphates

The phosphorylated condensed or hydrolysable tannins may also be dimeric or trimeric phosphates, consisting of one central phosphate group with two or three phenolic compounds attached to it. They may also be diphosphates or triphosphates, containing two or three phosphate groups bound to each other.

### Preferred compounds

Monoaromatic polyphenolic compounds such as pyrogallol or gallic acid show activity as antioxidants.

However, more complex polyphenols containing at least two different and/or the same aromatic rings show a much broader range of activity and are therefore preferred. For instance, these more complex compounds are excellent complexing agents for metals and proteins, and can act as antibacterials and/or antiallergens. These properties can be exclusively attributed to these more complex polyphenols.

More complex polyphenols are also active as antioxidants and radical scavengers, and possess a higher activity in the field than the monomeric substances. This has several reasons. First of all, one observes that the more conjugation, the better the oxidant. Secondly, these compounds also avoid radical formation by complexing metals.

For all these reasons, polyphenols as defined above are thus preferred above simple phenolic compounds.

The most preferred compounds are high molecular weight tannins, such as Chinese gallnut tannins, Tara tannins, grape seed tannins or other wine polyphenols, tea tannins or kaki tannins. All these natural product extracts possess average molecular weights of > 500.

Most preferred compounds are also single components of these tannins, namely 1,2,3,4,6-pentagalloylglucose, 1,3,4,5-tetragalloylquinic acid, catechine, epicatechine, gallocatechine and epigallocatechine.

### Reactivity

All the polyphenolic antioxidants described above have in common that at least one hydroxyl group is acidic enough to be deprotonated in aqueous medium. It is this phenolate that subsequently will react to form a phosphate ester.

### The phosphorylating agent

A phosphate group is introduced onto these polyphenolic compounds by using phosphoryl halides with the general structure:

RₙPOX₃₋ₙ (IV) or RᵥPX_{w} (V)

Wherein n = 0, 1 or 2,v + w = 3 or 5 X is a chloride, bromide or iodide and R is an alkoxide with an alkyl chain of 1-8 carbon atoms, or O-Phenyl or O-benzyl.

Most preferably, the reagent or phosphorylating agent is phosphorous oxychloride (POCl₃).

The above reagent can be applied in a ratio varying from 0.5 equivalent to over 25 equivalents per mole of phenolic hydroxyl compound, depending on the structure of the phenolic antioxidant, and more specifically on the amount of hydroxyl groups present in the molecule. The degree of phosphorylation should be sufficient to obtain a satisfying product stability, and thus also depends on the requirements for the application. The person skilled in the art will know when and how to adapt the reaction conditions.

Fully phosphorylated products, where all aromatic hydroxyl functions are phosphorylated, also form part of this invention as they are still active in some fields of application such as in protein complexation.

### Reaction conditions

The suitable phosphorylation method comprises the steps of reacting a phosphoryl chloride with a polyphenol in aqueous medium using inorganic bases.

In the present invention, the reaction is advantageously carried out in aqueous medium, preferably in water or mixtures of water with harmless solvents that do not react fast with phosphoryl halides. Examples of such solvents are acetone and/or alcohols that are miscible with water such as methanol, ethanol, n-propanol, glycerol and isopropanol. A solvent may be added to increase solubility of the phenolic compound. Preferably, the reaction is carried out in water. The reaction is preferably carried out under inert atmosphere, such as nitrogen or argon.

The base used is an inorganic base such as a metal hydroxide, a carbonate, a phosphate or ammonia. The base can be added prior and/or during addition of the phosphorylating agent, i.e. the reagent.

Examples of suitable bases include sodium hydroxide, potassium hydroxide, sodium carbonate and/or potassium carbonate. Most preferred is sodium hydroxide.

The unsaturated hydroxyl compound - the phenolic and/or polyphenolic compound - is suspended or dissolved in the aqueous medium, and the pH of the mixture is increased to values between 5 and 13 depending on the acidity of the hydroxyl function. Preferably, the pH of the reaction mixture is kept between 7 and 11.

The reaction temperature may vary between 0°C and 100 °C, preferably between 10°C and 60°C, most preferably between 10°C and 40°C. The concentration should be as high as possible both to suppress hydrolysis and to improve economics. Preferably, 1 to 20 volumes of aqueous medium are used. Most preferred is the use of 2 to 10 volumes of water.

The reagent is added to the aqueous medium over a period of 10 minutes to 24 hours, preferably over 1 to 4 hours. The addition time depends on the scale of the reaction and on the cooling capacity of the vessel.

The reaction time may vary from 1 to 24 hours depending on the scale and exothermicity, and on the origin of the compound.

The degree of phosphorylation can be controlled using high-pressure liquid chromatography.

After the phosphorylation step, the reaction mixture may be acidified. Preferably, a pH of 2 - 7 is obtained by the addition of mineral acids such as hydrochloric acid, sulphuric acid, phosphoric acid, or by organic acids such as formic, acetic acid or citric acid.

### Purification and isolation

Purification of the phosphorylated product can be achieved by the removal of salts. Methods to remove salts include, inter alia, the use of ultrafiltration, membrane filtration, reversed osmosis or chromatographies such as straight phase chromatography, reversed phase chromatography, size exclusion chromatography or ion exchange techniques.

Purification can also include extraction of unreacted phenolic compounds into a suitable organic solvent.

If solvents are present in the aqueous product solution, these solvents are preferably removed, for example by distillation.

The product may be used in its metal salt form and/or in its acidic form, depending on the application.

The product obtained in aqueous solution can be used as such, can be concentrated by distillation, and/or can be dried by complete evaporation, spray drying or freeze drying.

The product may also be isolated by precipitation of the phosphate or by precipitation of metal salts of the phosphates, preferably as sodium, potassium, magnesium or calcium salts.

### Extension of the phosphorylation process to simpler polyphenols

The phosphorylation process as described can be applied to all of the above defined categories of complex polyphenols.

The phosphorylation process described can be applied to any flavonoid compound, more specifically to the flavonoid compounds corresponding to the general formulas (VII) and (VIII): 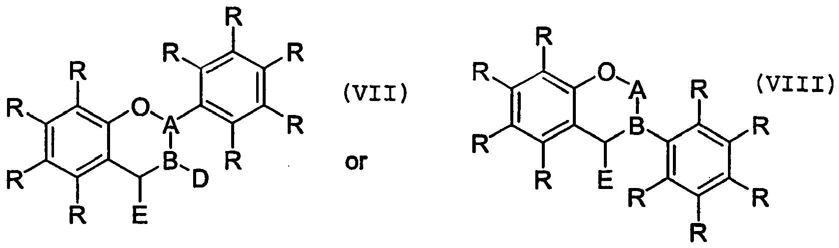 wherein:
- A and B are carbon atoms connected by a single or by a double bond
- D is hydrogen, hydroxide, or a hydroxide esterified with gallic acid or ellagic acid
- E is hydrogen, hydroxide, an double bonded oxygen, O-glucose or another condensed tannin corresponding to Formula (VII) or (VIII)
R is hydrogen, hydroxide, O-glucose, an O-alkyl group containing 1-3 carbon atoms or a phosphate group selected from the group consisting of OP(O) (OH)₂, OP(O) (OH)OP(O) (OH)₂, OP(O) (OH)OP(O) (OH)OP(O) (OH)₂ and a metal salt of such phosphate groups, with at least one of R being a phosphate group.

The phosphorylation process according to the invention (see infra) can also be applied to simple aromatic hydroxyl compounds containing one aromatic ring with at least one hydroxyl function. These phosphorylated compounds may also serve as stabilised antioxidants.

Such compounds include compounds corresponding to the general formula (VI): 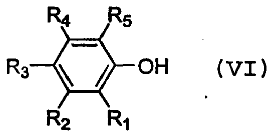 wherein:
- R₁, R₂, R₃, R₄, R₅ each independently of each other are hydrogen, hydroxide, carboxyl, Z, OZ, or COOZ, provided that at least one of the substituents R₁, R₂, R₃, R₄, R₅ is a hydroxyl or carboxyl group, Z being an alkyl chain consisting of 1-10 carbon atoms, or a CHCHCOOY group with Y = hydrogen, or an alkyl group containing 1-4 carbon atoms.

Examples with two hydroxyl groups are catechol, pyrogallol, guaiacol, resorcinol, 1,4-hydroquinone.

Examples with 3 hydroxyl groups are pyrogallol and hydroxyacids and their corresponding esters such as gallic acid, methyl gallate, ethyl gallate, propyl gallate, octyl gallate.

Compounds where one Rᵢ-group is a vinylic acid or ester are called hydroxycinnamic acids or esters. Examples thereof are p-coumaric acid, caffeic acid, ferulic acid, sinaptic acid, chlorogenic acid, curcumins and analogues in which the carboxylic acid group is esterified with a simple alcohol with 1-10 carbon atoms.

Another such compound is a phosphorylated stilbene or phloroglucinol with at least one covalently bound phosphate group selected from the group consisting of OP(O) (OH)₂, OP(O) (OH)OP(O) (OH)₂, OP(O) (OH)OP(O) (OH)OP(O) (OH)₂ and a metal salt of such phosphate groups. Typical examples thereof are the isomers of resveratrol.

Specific compounds or groups of compounds are phosphorylated forms of: catechol and derivatives thereof, such as DL-3,4-dihydroxyphenylalanine or DL-DOPA; catecholamines such as 3-hydroxytyramine or dopamine; phloroglucinol; phenolic acids, such as caffeic acid, dihydrocaffeic acid, ferulic acid, protocatechuic acid, chlorogenic acid, isochlorogenic acid, gentisic acid, homogentisic acid, gallic acid, hexahydroxydiphenic acid, ellagic acid, rosmarinic acid or lithospermic acid, nordihydroguaiaretic acid, and derivatives thereof, such as esters or their heterosides; arbutin, curcumin, tetrahydrocurcumin; salicylic acid, polyhydroxylated coumarins, polyhydroxylated lignans or neolignans; silymarin, apigenol, luteolol, quercetin, quercetagin, quercetagetin, chrysin, myricetin, rhamnetin, genistein, morin, gossypetin, kaempferol, rutin, naringin, narigenin, hesperitin, hesperidin, diosmin, diosmoside, amentoflavone, fisetin, vitexin, isoliquirtigenin, hesperidin methylchalcone, taxifoliol, silybin, silychristin, silydianin, catechin, epicatechin, gallocatechin, catechin gallate, gallocatechin gallate, epicatechin gallate, epigallocatechin gallate and epigallocatechin; glucogallin; proanthocyanidin; propyl gallate, isoamyloctyl gallate and dodecyl gallate; penta-O-galloyl glucose; tannic acid; various tannins such as gallotannin, ellagitannin; resveratrol (3, 4', 5'-trihydroxystilbene); and any derivatives or analogues of the foregoing compounds.

### Analysis

### Control of the phosphorylation degree

The phosphorylation reaction itself can be followed by chromatographic techniques such as thin layer chromatography or high-pressure liquid chromatography.

In the case of well-defined substrates, the degree of phosphorylation can be quantified by high-pressure liquid chromatography. In the case of complex mixtures of natural products, the reaction itself can be followed by chromatography, and the quantification of the amount of covalently bound phosphate groups can be executed by full hydrolysis of the product followed by a standard phosphate analysis.

Alternatively, the amount of phosphate groups attached can be quantified by ICP (inductively coupled plasma), leading to the total amount of phosphorus present in the molecule.

### Final product analysis

It is important for a well-defined compound that a covalent bond forms between the reactant and the phosphate group. The degree of phosphorylation may be measured as mentioned above.

An indirect proof for the presence of such bond can be obtained by a comparison of the free phosphate analysis in the final product before and after complete hydrolysis. The difference between these two values gives the amount of phosphate groups covalently attached to the phenolic compound, preferably a polyphenolic compound. The covalent bond can also be proven if the original compound is restored. The covalent bond between the hydroxyl containing compound and a phosphate group can also be demonstrated by NMR-techniques (both ¹³C-NMR and ³¹P-NMR).

### Properties testing

The antioxidant activity of the phosphorylated phenols or polyphenols can be measured by the Trolox-methodology, a test that is generally applicable for a wide range of antioxidants (Re et al. (1999), Free Radical Biology and Medicine 62:1231-1237). The test is based on the degree of reaction between the antioxidant and a coloured radical. The radical used is ABTS⁺, or [2,2-azinobis-(3-ethylbenzothiazoline-6-sulfonic acid)] radical cation. After contacting the antioxidant and the radical, the colour of the solution is spectrophotometrically measured. The data are compared relative to the activity of Trolox^{®} (6-hydroxy-2,5,7,8-tetramethylchroman-2-carboxylic acid).

Both acute and/or long-term antioxidant activity can be measured with this test, by varying the contact time between the antioxidant and the radical.

Phosphorylated tannins combined with an Fe(III)-solution react immediately to form a dark-blue, insoluble precipitate. This proves that the metal complexating properties of the naturally occurring tannin are at least partially retained in the phosphorylated product.

The rate of discolouration of the products obtained can be measured using various techniques. The easiest technique is to expose the product to direct sunlight, evaluating the discolouration rate by comparing non-phosphorylated polyphenol with phosphorylated polyphenol.

A more standardized method is to expose both products to a Heraeus Suntest CPS equipped with a xenonlamp for 1-72 hours.

### Possible uses of the phosphorylated compounds according to the invention

Phenols and/or polyphenols can be used in a wide variety of applications, but their use is often limited due to discolouration of the material on which the polyphenols are applied. This disadvantage of regular polyphenols can be avoided by using phosphorylated phenols and/or polyphenols in a number of applications.

One of the most important properties of phosphorylated polyphenols is their antioxidant activity. Antioxidants work through different pathways. They can prevent free radical formation by chelating redox-active metals, or they can neutralise radicals by reacting with these radicals to form stable components. Polyphenols have the advantage that they exhibit both mechanisms. They are active as radical scavengers.

For example, phosphorylated polyphenols can be used as antioxidants in such textile applications as the manufacture of swimwear or carpets, where the presence of efficient antioxidants is required to provide protection against oxidative bleaching agents such as aqueous hypochlorite solutions, hydrogen peroxide or ozone. The mentioned compounds and end-applications are provided as examples and are not intended to be limiting. Phosphorylated polyphenols can further be used as anti-oxidants in food applications.

Phosphorylated polyphenols such as tannins or syntans can be used in the textile industry to improve wash fastness by preventing dye migration out of the fibre or to improve the anti-staining properties of carpets and curtains. This last application is due to the complex formation of phosphorylated polyphenols with textile fibres such as polyamides or wool. The mentioned compounds are provided as examples and are not intended to be limiting.

Phosphorylated polyphenols such as tannins or syntans can be used in the textile industry to produce activated flock. More specific finishes can be prepared for fibres such as polyamide, viscose, polyester, cotton, aramide and polyolefins which will render the flock of these fibres active in an electrostatic field and also prevent the flock from adhering together in packages and lumps, thus providing free flowing properties during the flock application process.

Unlike the activation processes using non-modified tannins known to those skilled to the art, the use of phosphorylated polyphenols such as tannins or syntans impart significantly improved resistance against discolouration. It was also found that sift behaviour of thus produced activated flock was significantly better, resulting in lower levels of waste. Furthermore, the use of phosphorylated polyphenols such as tannins or syntans in this application allows for significant cost reductions by using less water and energy, reduction of process time and elimination of softeners from the activation recipe.

The preparation of activated flock using phosphorylated polyphenols such as tannins or syntans involves a wet process comprising a number of additions of different chemicals. This process is carried out at elevated temperatures typically in between 30° and 100°C, preferably between 50° and 80°C, and more preferably between 55° and 70°C. Multi-bath setups can be used, but preferably a 2 bath or 1 bath process will be used. Liquor ratios are typically in between 1:5 and 1:40, preferably between 1:10 and 1:30, and more preferably between 1:15 and 1:25.

Ordinary tap water can be used but water purified using one of the following techniques is preferred: ion exchange, distillation, reverse osmosis or other similar purification techniques known to those skilled in the art. The activation process is best carried out at pH in between 1 and 8, preferably between 2 and 6, and more preferably in between 3 and 5. To adjust pH organic or inorganic acids such as acetic, formic, oxalic, tartaric, sulphuric, hydrochloric acid can be used. The mentioned compounds are provided as examples and are not intended to be limiting.

In a first step a multivalent metal salt is added to the water/flock slurry. Typically aluminium, tin, zirconium or antimony salts are used, although other multivalent metals are not excluded from the current invention. Examples of metal salts used for this purpose include aluminium sulphate, aluminium chloride, potassium aluminium sulphate, tin(II) chloride, tin(II) sulphate, zirconium oxychloride, zirconium chloride, zirconium sulphate, zirconium acetate, potassium antimonyl tartrate. The mentioned compounds are provided as examples and are not intended to be limiting. Dosage levels are typically in between 0.1 and 3 % omf (on mass fabric), preferably between 0.25 and 2.5 % omf and more preferably between 0.5 and 2.0 % omf.

In a second addition step phosphorylated polyphenols such as tannins or syntans are added using dosage levels in between 0.25 and 7.0 % omf, preferably in between 0.5 and 5.0 % omf, and more preferably between 1.0 and 4.0 % omf. A number of phosphorylated polyphenols can be used to this purpose: phosphorylated syntans or condensed tannins can be used, but preferably phosphorylated hydrolysable tannins should be used in order to obtain best performance and colour stability. As the degree of phosphorylation is increased, increasing colour stability is imparted to the activated flock. To those skilled in the art it is easy to establish an optimum phosphorylation ratio versus performance.

In an optional third step a multivalent metal salt can be added. Typically aluminium, tin, zirconium or antimony salts are used, although other multivalent metals are not excluded from the current invention. Examples of suitable metal salts include aluminium sulphate, aluminium chloride, potassium aluminium sulphate, tin(II) chloride, tin(II) sulphate, Zirconium oxychloride, zirconium chloride, zirconium sulphate, zirconium acetate, potassium antimonyl tartrate. The mentioned compounds are provided as examples and are not intended to be limiting. Dosage levels are typically in between 0.1 and 3 % omf (on mass fabric), preferably between 0.25 and 2.5 % omf and more preferably between 0.5 and 2.0 % omf.

In a final addition an electrolyte and optionally a softener can be added. Suitable electrolytes include sulphates, chlorides, bromides, acetates, phosphates, nitrates of ammonium, sodium, potassium, lithium, magnesium or calcium. More specifically ammonium sulphate, sodium sulphate or sodium chloride can be used. The mentioned compounds are provided as examples and are not intended to be limiting. Typical dosage levels are in between 2 and 20 g/l bath liquor, preferably between 4 and 15 g/l and more preferably between 6 and 10 g/l.

Optionally a softener can be added; cationic, anionic, non-ionic or amphoteric softeners can be suitable, but anionic softeners are preferred. Suitable softeners can belong to any of the following chemical classes: alkyl sulphates, alkylether sulphates, sulphated alkylphenol ethoxylates, sulphonated esters, sulphonated hydrocarbons, alkyl phosphates, alkylether phosphates, quaternary ammonium salts and fatty acid ester derivatives of glycerol or amino acids. Examples of commercially available softeners suitable for this purpose: include Softifloc NF2, Ceranine VR or Cirrasol XLN. These, softeners are provided as examples and are not intended to be limiting.
Dosage levels depend on the active ingredient concentration in the commercially available product but typically are situated in between 0 and 10 % omf, preferably between 0 and 7.5 % omf and more preferably between 0 and 5.0 % omf.

The above-mentioned cascade of process steps can be applied in a multiple bath set-up (e.g. a different bath for every step) or in a single bath application. If a different bath is used for every process step the recycling of bath liquors is possible. If no liquors are recycled a 2-bath set-up in which the electrolyte and softener addition are combined in a second bath or a 1-bath process are the preferred embodiments.

Between different additions of chemicals the water/flock slurry is thoroughly mixed using techniques known to those skilled in the arts. Stirring times are typically in between 5 and 30 minutes, and preferably in between 10 and 20 minutes.
After completion of the wet activation process, as explained in the previous paragraphs, the activated flock fibres are separated from the processing liquor and dried according to techniques known to those skilled in the art.

In a further embodiment the multivalent metal salts can be replaced by one of the following agents: starch and derivatives (e.g. quaternized starch), cyclodextrines and derivatives, proteins such as casein, gelatine, soy-protein or enzymes.

The above provided processes impart significant improvements with respect to performance:
- better risetime
- improved sift behaviour generating less waste
- more hydrophilic
- better touch properties and softness

And cost assuming a production process as explained in example 11 is used:
- water consumption is reduced by 50 %
- waste water production is reduced by 50 %
- energy consumption is reduced by approx. 50 %
- process time is reduced by 40-50 %
- raw material cost is reduced due to the elimination of the softener.

Phosphorylated tannins can also be used in anti-allergenic compositions for people allergic for instance to mites or pets such as cats and dogs. These sources of allergens are provided as examples and are not intended to be limiting. Some people have an allergic reaction to proteins released by mites living in curtains, carpets or other textiles. Tannins complexate these proteins and thus prevent people from having the allergic reaction. However, in this application, staining of the treated textile due to tannin oxidation is an important issue. Use of phosphorylated polyphenols overcomes this drawback.

Colour-stable phosphorylated polyphenols can be used to treat leather. This application is based on the complex formation ability of polyphenols with proteins, applied in the leather manufacturing since ancient times. For high quality leather, that is only slightly coloured, phosphorylated polyphenols can avoid discolouration to happen.

Tannins are used in the production of tannates of active pharmaceutical ingredients (from now referred to as API's; e.g. ephedrine tannate, chlorpheniramine tannate, carbetapentane tannate). The above-mentioned API tannates are provided as examples and are not intended to be limiting. The use of tannin salts imparts certain advantages known to those skilled in the art. However it is also known that some of these compounds are fairly sensitive to oxidative degradations. The use of phosphorylated polyphenols in such applications therefore can render an improved stability against such degradation.

Tannins are also used in medical applications as anti-cancer agents or anti-viral agents. Phosphorylated tannins may be considered to be pro-drugs of the products described. However the use of naturally occurring tannins is limited due to their low bioavailability in vivo. By using pro-drugs such as phosphorylated tannins this problem can be alleviated.

The use of tannins, phosphoric acid and phosphates is well established in anti-corrosion applications such as phosphatation, passivation, wash primers, rust converters and anti-corrosion primers. In such applications the use of phosphorylated polyphenols such as tannins or syntans can have advantages because of the combined presence of the polyphenol moiety and phosphate group in one single molecule.

The use of phosphorus and phosphorus derivatives is well established in flame retarding compositions and is particularly important in intumescent flame retarding systems. The particular chemical nature of phosphorylated polyphenols such as tannins or syntans make these compounds particularly suitable for application in flame retardant applications.

Further applications of phosphorylated polyphenols can be found in the stabilisation, and as metal complexants.

### Examples

### Comp. Example 1: Phosphorylation of pyrogallol (TANFOS 167-103)

15.00 g. of pyrogallol was dissolved in 35 mL of water at room temperature. The solution was brought to a pH of 10 with NaOH 29 %, and 11.1 mL phosphorous oxychloride was added to the solution over a period of 2.5 hours. During this addition, more NaOH 29 % was added to maintain the pH at 10. After the addition, the reaction mixture was stirred over night, and analysed by HPLC.

Results (a/a %, HPLC):

| | |
|---|---|
| Pyrogallol: | 9.3 % |
| Pyrogallyl-1-phosphate: | 58.4 % |
| Pyrogallyl-2-phosphate: | 22.3 % |
| Pyrogallyl-diphosphate (Sum of both isomers): | 9.0 % |

### Comp. Example 2: Phosphorylation of gallic acid (TANFOS 167-113)

25.00 g. of gallic acid was dissolved in 150 mL of water at room temperature. The solution was brought to a pH of 10 with NaOH 29 %, and 16.1 mL of phosphorous oxychloride was added to the solution over a period of 2 hours. During this addition, more NaOH 29 % was added to maintain the pH at 10. After the addition, the reaction mixture was stirred for 2.5 hours, analysed by HPLC and acidified with HCl 25 % to a pH of 5.3.

Results (a/a %, HPLC):

| | |
|---|---|
| Gallic acid: | 9.0 % |
| Galloyl-3-phosphate: | 31.5 % |
| Galloyl-4-phosphate: | 38.9 % |
| Galloyl diphosphate: | 19.6 % |

### Example 3: Phosphorylation of Brewtan^{®} with 2 eq. of reagent (TANFOS 167-109)

100.0 g. of Brewtan^{®} (average molecular weight 1502 g/mole) was dissolved in 300 mL of water at room temperature. The solution was brought to a pH of 9.5 with NaOH 29 %, and 12.6 mL phosphorous oxychloride was added to the solution over a period of 3 hours. During this addition, more NaOH 29 % was added to maintain the pH at 9.5. After the addition, the reaction mixture was stirred overnight, and analysed by HPLC.

Results (a/a %, HPLC):

| | |
|---|---|
| Gallic acid: | 4.91 % |
| Galloyl-3-phosphate + Gallyl-4-phosphate: | 1.74 % |
| Galloyl diphosphate: | 0.15 % |
| Phosphorylated Brewtan: | 92.2 % |

The product was then acidified with HCl 25 % to a pH of 3.3, diluted with 200 mL of water and isolated via spray drying to obtain an off-white to slightly beige powder.

### Example 4: Phosphorylation of Brewtan^{®} with 9 eq. of reagent (TANFOS 167-117)

100.0 g of Brewtan^{®} (average molecular weight 1502 g/mole) was dissolved in 300 mL of water at room temperature under nitrogen atmosphere. The solution was adjusted to pH 9 with NaOH 29 %, and 55.85 mL phosphorous oxychloride was added to the solution over a period of 4 hours. During this addition, more NaOH 29 % was added to maintain the pH at 9. After the addition, the reaction mixture was stirred for 1 hour, and analysed by HPLC.

Results (a/a %, HPLC):

| | |
|---|---|
| Gallic acid: | 0.58 % |
| Galloyl-3-phosphate + Galloyl-4-phosphate: | 1.78 % |
| Galloyl diphosphate: | 1.57 % |
| Phosphorylated Brewtan: | 95.8 % |

The product was then acidified with HCl 25 % to a pH of 3.4, and isolated via spray drying to obtain an off-white to slightly beige powder.

### Example 5: Phosphorylation of grape seed tannin (TANFOS 167-134)

100.0 g. of grape seed tannin extracted by Ajinomoto OmniChem was dissolved in 300 mL of water at room temperature under nitrogen atmosphere. The solution was brought to a pH of 9 with NaOH 29 %, and 26.6 mL phosphorous oxychloride was added to the solution over a period of 1.5 hours. During this addition, more NaOH 29 % was added to maintain the pH at 9. After the addition, the reaction mixture was stirred overnight, and analysed by HPLC.

The product was then acidified with HCl 25 % to a pH of 3.1 and isolated via spray drying to obtain a pink powder.

### Example 6: Trolox antioxidant activity

*To prove that the phosphorylated products are still effective antioxidants*.

### Example 7: Colour improvement after phosphorylation

To test the colour stability of our products, both reference materials and phosphorylated samples were exposed to UV-irradiation by a xenon lamp in a Heraeus Suntest CPS. This setup standardizes to colour degradation as a function of time. All samples were irradiated for 42 hours. Following results were notified:

| Sample | Sample description | Results |
|---|---|---|
| TANFOS 134 | Fully phosphorylated grape seed extract | Beige to orange, almost no discolouration |
| Grape Seed tannin Ajinomoto OmniChem | Starting material TANFOS 134 | Terracotta red, severe discolouration |
| TANFOS 109 | Brewtan, 2 phosphate groups | Slightly beige |
| TANFOS 120 | Brewtan, 4 phosphate groups | Off-white, no discolouration |
| TANFOS 117 | Brewtan, 9 phosphate groups | Off-white, no discolouration |
| Brewtan^{®} 99 | Starting material for TANFOS 109, 117, 120 | Beige, discolouration |

### Example 8: Traditional flock activation using Floctan.

Polyamide 6.6 flock 0.9 dtex, 0.6 mm cutting length was scoured for 20 minutes in demineralised water at 60°C using a liquor ratio of 1:20 prior to activation.
Activation: 2-bath 4-steps process.
Bath 1: liquor ratio: 1:30
   Demineralised water is heated to 60°C
   Add 1 % omf Al₂(SO₄)₃.18Aq
   Add flock fibres
      Stir 10 minutes at 60°C
   Add 1.0 % omf Floctan 1
      Stir 10 minutes at 60°C
   Add 0.5 % omf Al₂(SO₄)3.18Aq
      Stir 10 minutes at 60°C
      Remove liquor by filtration
Bath 2: liquor ratio: 1:30
   Demineralised water is heated to 60°C
   Add 8 g/l (NH₄)₂SO₄
   Add flock fibres retrieved from bath 1
      Stir 20 minutes at 60°C
      Remove liquor by filtration
      Dry flock fibres at T≤60°C

The above-described process can be considered as representative for the processes currently applied around the world for the production of activated flock.

### Example 9: Flock activation using Tanfos.

Polyamide 6.6 Full Dull flock 1.9 dtex, 1.2 mm cutting length was scoured for 20 minutes in demineralised water at 60°C using a liquor ratio of 1:37 prior to activation.
Activation: 2-bath 4-steps process.
Bath 1: liquor ratio: 1:30
   Demineralised water is heated to 60°C
   Add 1 % omf Al₂(SO₄)₃.18Aq
   Add flock fibres
      Stir 10 minutes at 60°C
   Add 1.2 % omf Tanfos 167-120
      Stir 10 minutes at 60°C
   Add 0.5 % omf Al₂(SO₄)₃.18Aq
      Stir 10 minutes at 60°C
      Remove liquor by filtration
Bath 2: liquor ratio: 1:30
   Demineralised water is heated to 60°C
   Add 8 g/l (NH₄)₂SO₄
   Add flock fibres retrieved from bath 1
      Stir 20 minutes at 60°C
      Remove liquor by filtration
      Dry flock fibres at T≤60°C

### Flock properties:

| Properties after conditioning | Floctan 1 Ref (Example 8) | Tanfos 167-120 |
|---|---|---|
| Moisture content | 4.9 % | 4.8 % |
| | | |
| Mahlo | 84 | 82 |
| Risetime | 17 sec | 19 sec |
| Remarks | OK | OK |
| | | |
| Sift residue after 60 rotation | 45 % | 30 % |
| | | |
| Wetting | 1 min 30 sec | 2 sec |

The tabulated values clearly show the activated flock prepared with Tanfos 167-120 is superior to the reference material prepared with a non-modified hydrolysable tannin in respect to sift behaviour and wetting properties. Other properties are similar.

In order to evaluate colour stability flocked samples were prepared. No significant difference in flock density between the reference and Tanfos 190-102 treated samples was found.

| Colour stability | Floctan 1 Ref (Example 8) | Tanfos 167-120 |
|---|---|---|
| 30 min @ 150°C - electrical oven CieL*a*b dE | 6.6 | 3.9 |
| | | |
| 4 hours saturated NH₃ atmosphere | 8.4 | 4.5 |
| | | |
| 184 hours Xenon arc exposure | 5.0 | 1.8 |
| | | |
| 19 days real time ageing | 6.4 | 2.5 |

All colour measurements were done in triplicate and then averaged. The tabulated dE values refer to the colour difference found on samples after stressing, using the colour prior to the stress test as a reference.

Real time ageing experiments were carried out in a dedicated smoking area oriented south, guaranteeing high levels of exposure to cigarette smoke and sun light.

The tabulated values clearly show that the flock activated with a phosphorylated hydrolysable tannin (Tanfos 167-120) has a significantly improved colour fastness compared to the material activated using a non-modified but otherwise similar hydrolysable tannin (Floctan 1).

### Example 10: Flock activation using Tanfos - without softener.

Polyamide 6.6 flock 0.9 dtex, 0.6 mm cutting length was scoured for 20 minutes in demineralised water at 60°C using a liquor ratio of 1:20 prior to activation.
Activation: 2-bath 4-steps process.
Bath 1: liquor ratio: 1:30
   Demineralised water is heated to 60°C
   Add 1 % omf Al₂(SO₄)₃.18Aq
   Add flock fibres
      Stir 10 minutes at 60°C
   Add 1.2 % omf Tanfos 167-120
      Stir 10 minutes at 60°C
   Add 0.5 % omf Al₂(SO₄)₃.18Aq
      Stir 10 minutes at 60°C
      Remove liquor by filtration
Bath 2: liquor ratio: 1:30
   Demineralised water is heated to 60°C
   Add 8 g/l (NH₄)₂SO₄
   Add flock fibres retrieved from bath 1
      Stir 20 minutes at 60°C
      Remove liquor by filtration
      Dry flock fibres at T≤60°C

A reference sample was prepared according to the same process using 1.0 % omf Floctan 1 instead of Tanfos 167-120. In the second bath additionally 0.8 % omf Softifloc NF2 was used as a softener in the reference sample.

### Flock properties:

| Properties after conditioning | Floctan 1 Ref | Tanfos 167-120 |
|---|---|---|
| Moisture content | 3.4 % | 3.5 % |
| | | |
| Mahlo | 70 | 71 |
| Risetime | 18 sec | 13 sec |
| Remarks | Slows down, | OK - no |
| | onion peel | remarks |
| | | |
| Sift residue after 60 rotation | 17 % | 4 % |
| | | |
| Wetting | 20 sec | <1 sec |
| | | |
| Touch properties | | Less crispy than ref. Softer touch than ref. |

The tabulated values clearly show the activated flock prepared with Tanfos 167-120 is in many aspects superior to the reference material prepared with a non-modified hydrolysable tannin:
- Better risetime
- improved sift behaviour generating less waste
- more hydrophilic
- better touch properties and softness

In order to evaluate colour stability flocked samples were prepared. No significant difference in flock density between the reference and Tanfos 190-102 treated samples was found.

| Colour stability | Floctan 1 Ref | Tanfos 167-120 |
|---|---|---|
| 30 min @ 150°C - electrical oven CieL*a*b dE | 6.8 | 4.4 |
| | | |
| 4 hours saturated NH₃ atmosphere | 4.5 | 2.5 |
| | | |
| 137 hours Xenon arc exposure | 6.1 | 2.8 |

All colour measurements were done in triplicate and then averaged. The tabulated dE values refer to the colour difference found on samples after stressing, using the colour prior to the stress test as a reference.

The tabulated values clearly show that the flock activated with a phosphorylated hydrolysable tannin (Tanfos 167-120) has a significantly improved colour fastness compared to the material activated using a non-modified but otherwise similar hydrolysable tannin (Floctan 1).

### Example 11: Flock activation using Tanfos - cost efficient process.

Polyamide 6.6 flock 0.9 dtex, 0.6 mm cutting length was scoured for 20 minutes in demineralised water at 60°C using a liquor ratio of 1:20 prior to activation.
Activation: 1-bath 3-step process.
Bath 1: liquor ratio: 1:30
   Demineralised water is heated to 60°C
   Add 8 g/l (NH₄)₂SO₄
   Add 1 % omf Al₂ (SO₄)₃.18Aq
   Add flock fibres
      Stir 10 minutes at 60°C
   Add 1.2 % omf Tanfos 167-120
      Stir 10 minutes at 60°C
   Add 0.5 % omf Al₂(SO₄)₃.18Aq
      Stir 10 minutes at 60°C
      Remove liquor by filtration
      Dry flock fibres at T≤60°C

This process provides significant cost reduction compared to the process described in Example 8 assuming no scouring is used as is usually the case in industrial practice:
- water consumption is reduced by 50 %
- waste water production is reduced by 50 %
- energy consumption is reduced by approx. 50 %
- process time is reduced by 40-50 %
- raw material cost is reduced due to the elimination of the softener.

## Claims

1. A phosphorylated condensed tannin according to Formula (III) 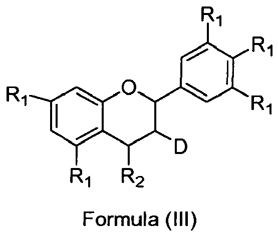 wherein
- R₁ is hydrogen, hydroxide, O-glucose, an O-alkyl group containing 1-3 carbon atoms or a phosphate group selected from the group consisting of OP(O)(OH)₂, OP(O) (OH)OP(O) (OH)₂, OP(O)(OH)OP(O)(OH)OP(O)(OH)₂ and a metal salt of such phosphate groups, with at least one of R₁ being a phosphate group.
- D is hydroxide, O-glucose or a galloyl residue with 0-3 phosphate groups as defined for R₁
- R₂ is a hydrogen or another component of Formula (III)

2. A phosphorylated condensed tannin corresponding to the formula 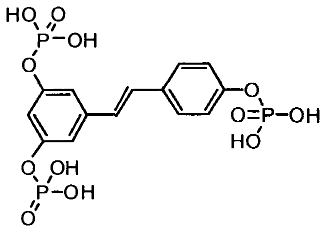 or a salt thereof.

3. A phosphorylated hydrolysable tannin comprising at least one covalently bound phosphate group selected from the group consisting of OP(O)(OH)₂, OP(O)(OH)OP(O)(OH)₂ and OP(O)(OH)OP(O)(OH)OP(O)(OH)₂ or a metal salt of such phosphate groups.

4. A phosphorylated hydrolysable tannin as in claim 3 wherein the hydrolysable tannin is 1,2,3,4,6-pentagalloylglucose.

5. A phosphorylated tannin according to any of the preceding claims, wherein the tannin to phosphorylate is isolated from natural sources.

6. Use of phosphorylated tannins according to any of claims 1 to 5 for the preparation of activated flock used in textile flocking.

7. Use of phosphorylated tannins as in claim 6 in a single bath system.

8. Use of phosphorylated tannins according to any of claims 1 to 5 as carriers for pharmaceutical products.

9. Use of a phosphorylated condensed tannin comprising at least one covalently bound phosphate group selected from the group consisting of OP(O) (OH)₂, OP(O) (OH)OP(O) (OH)₂ and OP(O) (OH)OP(O) (OH)OP(O) (OH)₂ or a metal salt of such phosphate groups for the preparation of activated flock used in textile flocking.

10. Use of a phosphorylated condensed tannin as in claim 8 in a single bath system.

11. Use of a phosphorylated condensed tannin corresponding to the general formula: 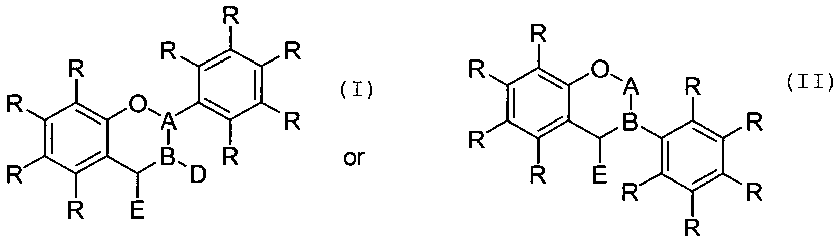 wherein:
- A and B are carbon atoms connected by a single or by a double bond
- D is hydrogen, hydroxide, or a hydroxide esterified with gallic acid or ellagic acid
- E is hydrogen, hydroxide, O-glucose or another condensed tannin corresponding to Formula (I) or (II)
- R is hydrogen, hydroxide, O-glucose, an O-alkyl group containing 1-3 carbon atoms or a phosphate group selected from the group consisting of OP(O) (OH)_{2,} OP(O) (OH)OP(O) (OH)₂, OP(O) (OH)OP(O) (OH)OP(O) (OH)₂ and a metal salt of such phosphate groups, with at least one of R being a phosphate group,
for the preparation of activated flock used in textile flocking.

12. Use of a phosphorylated condensed tannin as in claim 11 in a single bath system.

## Patentansprüche

1. Phosphoryliertes kondensiertes Tannin der Formel (III) 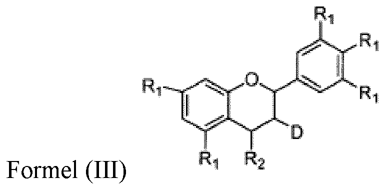 wobei
- R₁ Wasserstoff, Hydroxid, O-Glukose, eine O-Alkylgruppe mit 1 bis 3 Kohlenstoffatomen oder eine Phosphatgruppe, ausgewählt aus der Gruppe bestehend aus OP(O) (OH)₂, OP(O) (OH)OP(O) (OH)₂, OP(O) (OH)OP(O) (OH)OP(O) (OH)₂, und ein Metallsalz solcher Phosphatgruppen ist, wobei zumindest eines von R₁ eine Phosphatgruppe ist;
- D Hydroxid, O-Glukose oder ein Galloylrest mit 0 bis 3 Phosphatgruppen, wie in Bezug auf R₁ definiert ist,
- R₂ ein Wasserstoff oder eine andere Komponente der Formel (III) ist.

2. Phosphoryliertes kondensiertes Tannin der Formel 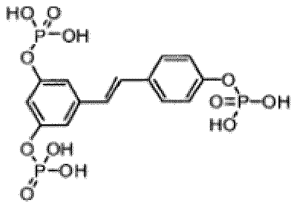 oder ein Salz davon.

3. Phosphoryliertes hydrolysierbares Tannin, umfassend zumindest eine kovalent gebundene Phosphatgruppe, ausgewählt aus der Gruppe bestehend aus OP(O) (OH)₂, OP(O)(OH)OP(O)(OH)₂, OP(O)(OH)OP(O)(OH)OP(O)(OH)₂, oder ein Metallsalz solcher Phosphatgruppen.

4. Phosphoryliertes hydrolysierbares Tannin nach Anspruch 3, wobei das hydrolysierbare Tannin 1,2,3,4,6-Pentagalloylglukose ist.

5. Phosphoryliertes Tannin nach einem der vorstehenden Ansprüche, wobei das zu phosphorylierende Tannin aus natürlichen Quellen isoliert ist.

6. Verwendung von phosphorylierten Tanninen nach einem der Ansprüche 1 bis 5 zur Herstellung eines aktivierten Flocks, der bei der Textilbeflockung Anwendung findet.

7. Verwendung von phosphorylierten Tanninen nach Anspruch 6 in einem Ein-Bad-System.

8. Verwendung von phosphorylierten Tanninen nach einem der Ansprüche 1 bis 5 als Träger für pharmazeutische Produkte.

9. Verwendung eines phosphorylierten kondensierten Tannins, umfassend zumindest eine kovalent gebundene Phosphatgruppe, ausgewählt aus der Gruppe bestehend aus OP(O) (OH)₂, OP(O) (OH)OP(O)(OH)₂ und OP(O) (OH)OP(O)(OH)OP(O)(OH)₂, oder ein Metallsalz solcher Phosphatgruppen, zur Herstellung eines aktivierten Flocks, der bei der Textilbeflockung Anwendung findet.

10. Verwendung eines phosphorylierten kondensierten Tannins nach Anspruch 8 in einem Ein-Bad-System.

11. Verwendung eines phosphorylierten kondensierten Tannins der allgemeinen Formel: 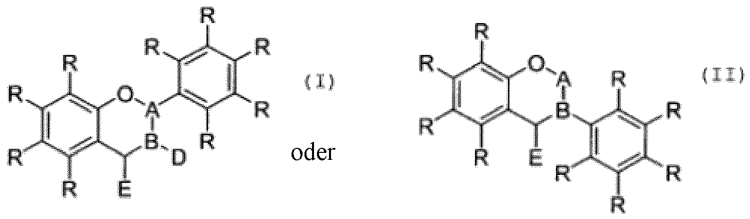 wobei:
- A und B Kohlenstoffatome sind, verbunden mittels Einfach- oder Doppelbindung
- D Wasserstoff, Hydroxid oder ein Hydroxid, verestert mit Gallussäure oder Ellagsäure, ist
- E Wasserstoff, Hydroxid, O-Glukose oder ein anderes kondensiertes Tannin der Formel (I) oder (II) ist
- R Wasserstoff, Hydroxid, O-Glukose, eine O-Alkylgruppe mit 1 bis 3 Kohlenstoffatomen oder eine Phosphatgruppe, ausgewählt aus der Gruppe bestehend aus OP(O) (OH)₂, OP(O) (OH)OP(O) (OH)₂, OP(O) (OH)OP(O) (OH)OP(O) (OH)₂, und ein Metallsalz solcher Phosphatgruppen ist, wobei zumindest einfach von R eine Phosphatgruppe ist,
zur Herstellung eines aktivierten Flocks, der bei der Textilbeflockung Anwendung findet.

12. Verwendung eines phosphorylierten kondensierten Tannins nach Anspruch 11 in einem Ein-Bad-System.

## Revendications

1. Tanin condensé phosphorylé répondant à la formule (III) 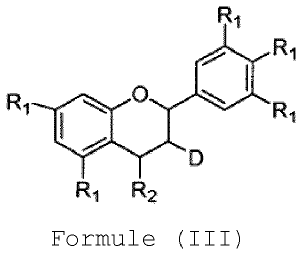 où
- R₁ est un hydrogène, un hydroxyde, un O-glucose, un groupe O-alkyle contenant 1 à 3 atomes de carbone ou un groupe phosphate choisi parmi le groupe constitué par OP(O) (OH)₂, OP(O)(OH)OP(O)(OH)₂, OP(O)(OH)OP(O)(OH)OP(O)(OH)₂ et un sel métallique de tels groupes phosphate, au moins un R₁ étant un groupe phosphate,
- D est un hydroxyde, un O-glucose ou un résidu de galloyle ayant 0-3 groupes phosphate tels que définis pour R₁ ;
- R₂ est un hydrogène ou un autre composant de la formule (III) .

2. Tanin condensé phosphorylé correspondant à la formule : 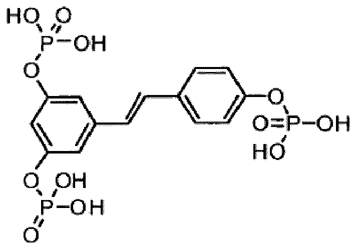 ou un sel de celui-ci.

3. Tanin hydrolysable phosphorylé comprenant au moins un groupe phosphate à liaison covalente choisi parmi le groupe constitué par OP(O)(OH)₂, OP(O)(OH)OP(O)(OH)₂ et OP(O)(OH)OP(O)(OH)OP(O)(OH)₂ ou un sel métallique de tels groupes phosphate.

4. Tanin hydrolysable phosphorylé selon la revendication 3, dans lequel le tanin hydrolysable est le 1,2,3,4,6-pentagalloylglucose.

5. Tanin phosphorylé selon l'une quelconque des revendications précédentes, dans lequel le tanin devant être phosphorylé est isolé à partir de sources naturelles.

6. Utilisation de tanins phosphorylés selon l'une quelconque des revendications 1 à 5 pour la préparation d'un floc activé utilisé dans le flocage textile.

7. Utilisation de tanins phosphorylés selon la revendication 6 dans un système à un seul bain.

8. Utilisation de tanins phosphorylés selon l'une quelconque des revendications 1 à 5 en tant que vecteurs pour produits pharmaceutiques.

9. Utilisation d'un tanin condensé phosphorylé comprenant au moins un groupe phosphate à liaison covalente choisi parmi le groupe constitué par OP(O) (OH)₂, OP(O)(OH)OP(O)(OH)₂ et OP(O)(OH)OP(O)(OH)OP(O)(OH)₂ ou un sel métallique de tels groupes phosphate pour la préparation d'un floc activé utilisé dans le flocage textile.

10. Utilisation d'un tanin condensé phosphorylé selon la revendication 8 dans un système à un seul bain.

11. Utilisation d'un tanin condensé phosphorylé correspondant à la formule générale : 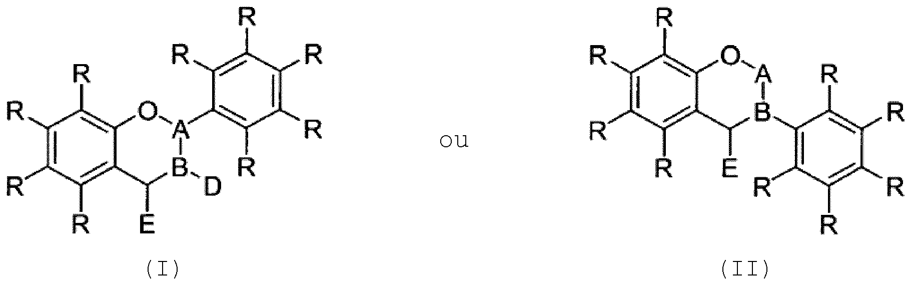 où :
- A et B sont des atomes de carbone connectés par une liaison simple ou double,
- D est un hydrogène, un hydroxyde, ou un hydroxyde estérifié avec de l'acide gallique ou de l'acide ellagique,
- E est un hydrogène, un hydroxyde, un O-glucose ou un autre tanin condensé correspondant à la formule (I) ou (II),
- R est un hydrogène, un hydroxyde, un O-glucose, un groupe O-alkyle contenant 1 à 3 atomes de carbone ou un groupe phosphate choisi parmi le groupe constitué par OP(O) (OH)₂, OP(O)(OH)OP(O)(OH)₂, OP(O)(OH)OP(O)(OH)OP(O)(OH)₂ et un sel métallique de tels groupes phosphate, au moins un R étant un groupe phosphate,
pour la préparation d'un floc activé utilisé dans le flocage textile.

12. Utilisation d'un tanin condensé phosphorylé selon la revendication 11 dans un système à un seul bain.
